# EUROPEAN PATENT SPECIFICATION

(11) **EP 2 809 319 B1**
(45) Date of publication and mention of the grant of the patent: **18.04.2018**
(21) Application number: 13744046.7
(22) Date of filing: 01.02.2013
(51) Int. Cl.: A61K 31/366, A61K 31/22, A61K 31/404, A61K 31/40, A61P 27/02

(54) **IMPROVEMENTS IN TEAR FILM STABILITY**
VERBESSERUNGEN AN TRÄNENFILMSTABILITÄT
AMÉLIORATIONS DANS LA STABILITÉ DU FILM LACRYMAL

(30) Priority: 02.02.2012 AU 2012900383
(43) Date of publication of application: 10.12.2014
(73) Proprietor: The University of Sydney, NSW 2006 (AU); Ooi, Kenneth Gek-Jin, Randwick, NSW 2031 (AU)
(72) Inventor: OOI, Kenneth Gek-Jin, Randwick New South Wales 2031 (AU); WATSON, Stephanie Louise, Queens Park New South Wales 2022 (AU)
(74) Representative: Titmus, Craig Edward
(86) International application number: PCT/AU2013/000087
(87) International publication number: WO 2013/113067

(56) References cited:
- WO-A1-2005/105069
- US-A1- 2010 197 735
- US-A1- 2010 239 518
- US-A1- 2010 247 583
- US-A1- 2011 021 974
- US-A1- 2011 052 678
- GEGG MATTHEW E ET AL: "Suppression of autoimmune retinal disease by lovastatin does not require Th2 cytokine induction", JOURNAL OF IMMUNOLOGY, vol. 174, no. 4, 15 February 2005 (2005-02-15), pages 2327-2335, XP002742267, ISSN: 0022-1767
- MARCANO J C ET AL: "Reduction in Nitric Oxide Aqueous Humor Levels After Treatment With Atorvastatin During Endotoxin-induced Uveitis in Rabbits.", ARVO ANNUAL MEETING ABSTRACT SEARCH AND PROGRAM PLANNER, vol. 2002, 2002, XP009185333, & ANNUAL MEETING OF THE ASSOCIATION FOR RESEARCH IN VISION AND OPHTHALMOLOGY; FORT LAUDERDALE, FLORIDA, USA; MAY 05-10, 2002
- DATABASE WPI Derwent Publications Ltd., London, GB; AN 2006-463920, XP055082661 & WO 2006 064825 A1 (SANTEN PHARMACEUTICAL CO. LTD.) 22 June 2006

## Description

### Field of the invention

The invention relates to treatment of ocular surface inflammatory disorders, in particular to treatment of disorders characterised by unstable tear film.

### Background of the invention

Reference to any prior art in the specification is not, and should not be taken as, an acknowledgment or any form of suggestion that this prior art forms part of the common general knowledge in Australia or any other jurisdiction or that this prior art could reasonably be expected to be ascertained, understood and regarded as relevant by a person skilled in the art.

The tear film, otherwise known as pre-corneal film is a liquid layer that bathes the cornea and conjunctiva. It creates a perfectly smooth liquid outer layer that polishes the corneal surface, mechanically traps and flushes out foreign bodies and chemicals, contains bacteriostatic substances that inhibit the growth of microorganisms, and reduces the surface friction associated with eyelid blinking and eye movement.

The tear film is generally understood to comprise three distinct layers, which, from the outermost surface are as follows: lipid layer; aqueous layer and mucous layer. The lipid layer also known as 'meibum' is understood to be a monolayer of lipid composed of a polar phase and a non polar phase.

The principal lipid components of meibum are phospholipids, sphingolipids, triglycerides, wax esters, cholesterol esters and free fatty acid hydrocarbons. Generally for all but the wax esters, the ester component of the relevant lipid is an acid. For wax esters, the ester component may be acid or alcohol.

The wax and sterol esters and hydrocarbons are generally regarded as non polar lipid components of meibum. Phospho- and sphingolipids are generally regarded as polar lipid components [McCulley JP and Shine WE 2002 Bioscience Reports 21: 407-418].

Meibum is generally understood to provide three essential functions. First, as a lubricant to facilitate the movement of eyelids during a blink. Second as a barrier to prevent evaporation of the aqueous layer. Third as a barrier to entry to micro-organisms and organic material.

It is generally understood that both the composition of meibum and the aqueous layer may be relevant to the functionality of the meibum [McCulley JP *supra*]*.* For example, the stability of tear film, otherwise known as tear film break up time, decreases where either the aqueous layer or meibum, or both are abnormal. However, the actual contribution of aqueous layer and meibum, or the principal components of both, to loss of functionality and disease is not precisely known.

Unusual non polar lipid compositions have been observed in individuals having functional and less functional meibum. As reported in McCulley *supra,* one study found normal and abnormal meibum contained cholesterol esters and unsaturated cholesterol and wax ester fatty acids. In contrast, other normal meibum had no cholesterol esters and no wax ester unsaturated fatty acids of alcohols. Further, where a disease state is present, unsaturation in polar lipid fatty acids is also present. Some have suggested that decreased amounts of polar lipids may result in destabilisation of the lipid tear layer, uneven spreading and increased aqueous tear evaporation [Jackson BW 2008 Can J Ophthalmol 43: 170-179.] Others have suggested that enzymolysis of either polar or non polar components of meibum by triglyceride lipase, cholesterol esterase and wax esterase could result in disruption of tear film integrity [Bron AJ et al. 2004 Ocul Surf 2:149-65]. In summary, the role of any one component of aqueous or meibum layers in terms of relative contribution to the formation of a functional meibum monolayer is not fully understood.

The formation of a functional monolayer is critical because, as mentioned, an unstable tear film having a decreased break up time is a characteristic feature of many forms of ocular surface inflammatory disorders. Posterior blepharitis and associated inflammation is one example.

Generally speaking, these disorders are managed by eye lid cleaning and related physical manipulations, by application of artificial tears or wetting agents, or by chemotherapy regimes that focus on managing the associated inflammation, for example with steroids, immunosuppressants, and where there is bacterial infection, antibiotics.

Gegg M et al. Journal of Immunology 174(4): 2327-2335 discusses that suppression of autoimmune retinal disease by lovastatin does not require Th2 cytokine induction. Marcano J et al. Arvo Annual Meeting Abstract Search and Program Planner vol 2002 & Annual Meeting of the Association for Research in Vision and Ophthalmology, Fort Lauderdale, Florida, USA: 10 May 2002 describes a reduction in nitric oxide aqueous humor levels after treatment with atorvastatin during endotoxin-induced uveitis in rabbits. WO 2005/105069 relates to a method of treating glaucoma, and the use of a composition formulated for ophthalmic administration comprising a HMG CoA reductase inhibitor selected from compactin, lovastatin, simvastatin, pravastatin, mevastatin, fluvastatin, rosuvastatin, atorvastatin, pitavastatin. cervistatin, berivastatin, dalvastatin and glenvastatin.

There remains a need for new approaches to stabilising tear films, or for improving the stability of tear films, especially in individuals having an ocular surface inflammatory disorder.

There is also a need for new approaches to treatment of ocular surface inflammatory disorders, especially posterior blepharitis or one or more conditions or symptoms generally associated with same.

### Summary of the invention

The invention seeks to address one or more of the above mentioned needs or limitations. The invention is defined according to the claims.

Thus, the invention provides a HMG CoA reductase inhibitor for use in a method of treating an ocular surface inflammatory disorder in an individual:
wherein the ocular surface inflammatory disorder is characterised by an unstable tear film;
wherein the HMG CoA reductase inhibitor is administered to the ocular surface of the individual;
wherein the HMG CoA reductase inhibitor reduces the synthesis of a cholesterol by a meibum-producing tissue thereby stabilising the tear film in the individual; and wherein the HMG CoA reductase inhibitor is a statin.

In one embodiment, the statin has a hexahydronaphthalene ring or a fluorophenyl ring.

In one embodiment, the statin is selected from lovastatin, simvastatin, pravastatin, fluvastatin, atorvastatin, rosuvastatin, pitavastatin, dalvastatin, glenvastatin, bervastatin, cerivastatin, or carvastatin; optionally atorvastatin.

In one embodiment, the atorvastatin is administered in an amount of 1 to 15 drops per day from a 50µM atorvastatin solution; and optionally the administration is for 1 to 4 weeks.

In one embodiment, the HMG CoA reductase inhibitor for use according to the invention further comprises an anti-inflammatory compound and/or an immunosuppressant. In one embodiment, the anti-inflammatory compound is a steroid and/or the immunosuppressant is a cyclosporin.

In one embodiment, the HMG CoA reductase inhibitor for use according to the invention is separately administered with an anti-inflammatory compound and/or an immunosuppressant. In one embodiment, the anti-inflammatory compound is a steroid and/or the immunosuppressant is a cyclosporin.

In one embodiment, the HMG CoA reductase inhibitor for use according to the invention further comprises an antibiotic.

In one embodiment, the disorder is posterior blepharitis.

The invention also provides a composition formulated for ophthalmic administration, comprising a HMG CoA reductase inhibitor and further comprising an anti-inflammatory compound and/or an immunosuppressant, wherein the HMG CoA reductase inhibitor is a statin, optionally wherein the HMG CoA reductase inhibitor is a atorvastatin.

Also disclosed is a method for stabilising a tear film, or for improving the stability of a tear film, or for increasing a tear film break up time, in an individual having an ocular surface inflammatory disorder. The method includes the following steps:
- providing an individual having an ocular surface inflammatory disorder that is characterised by an unstable tear film;
- providing a compound to an ocular surface of the individual to reduce the synthesis of a cholesterol by a meibum -producing tissue;
thereby stabilising the tear film, or improving the stability of the tear film, or increasing the tear film break up time in the individual.

Also disclosed is a method of stabilising a tear film in an individual having an ocular surface inflammatory disorder including the following steps:
- providing an individual having an ocular surface inflammatory disorder that is characterised by an unstable tear film;
- providing a compound to an ocular surface of the individual to reduce the synthesis of a cholesterol by a meibum -producing tissue;
- providing an anti -inflammatory compound and/or an immunosuppressant to an ocular surface of the individual;
thereby stabilising the tear film in the individual.

Also disclosed is a method of stabilising a tear film in an individual having an ocular surface inflammatory disorder including the following steps:
- providing an individual having an ocular surface inflammatory disorder that is characterised by an unstable tear film;
- providing a HMG CoA reductase inhibitor to an ocular surface of the individual to reduce the synthesis of a cholesterol by a meibum -producing tissue;
thereby stabilising the tear film in the individual.

Also disclosed is a method of treating posterior blepharitis in an individual including the following steps:
- providing an individual having posterior blepharitis;
- providing a therapeutically effective amount of a HMG CoA reductase inhibitor to an ocular surface of the individual;
thereby treating posterior blepharitis in the individual.

Also disclosed is a method of treating posterior blepharitis in an individual including the following steps:
- providing an individual having posterior blepharitis;
- providing a therapeutically effective amount of atorvastatin to an ocular surface of the individual;
thereby treating posterior blepharitis in the individual.

In another embodiment there is provided a composition formulated for ophthalmic administration including a compound for reducing the synthesis of a cholesterol by a meibum -producing tissue. In another embodiment there is provided a composition formulated for ophthalmic administration including a compound for the reduction of lipid from leaking blood vessels.

In another embodiment there is provided a composition formulated for ophthalmic administration including a therapeutically effective amount of a HMG CoA reductase inhibitor, for example, atorvastatin. The composition may further include a steroid such as described herein or an immunosuppressant.

In another embodiment there is provided a HMG CoA reductase inhibitor, preferably a statin, more preferably atorvastatin, for use in the treatment of posterior blepharitis.

Also described herein is use of a HMG CoA reductase inhibitor, preferably a statin, more preferably atorvastatin, in the manufacture of a medicament for the treatment of posterior blepharitis.

In another embodiment there is provided a use of a HMG CoA reductase inhibitor, preferably a statin, more preferably atorvastatin, to treat posterior blepharitis.

### Detailed description of the embodiments

Reference will now be made in detail to certain embodiments of the invention. While the invention will be described in conjunction with the embodiments, it will be understood that the intention is not to limit the invention to those embodiments. On the contrary, the invention is intended to cover all alternatives, modifications, and equivalents, which may be included within the scope of the present invention as defined by the claims.

One skilled in the art will recognize many methods and materials similar or equivalent to those described herein, which could be used in the practice of the present invention. The present invention is in no way limited to the methods and materials described.

It will be understood that the invention disclosed and defined in this specification extends to all alternative combinations of two or more of the individual features mentioned or evident from the text or drawings. All of these different combinations constitute various alternative aspects of the invention.

### A. Definitions

*'tear film',* also known as *'lacrimal layer, 'pre-ocular tear film', 'tear layer'* and *'pre-corneal film'* generally refers to a composition covering the anterior surface of the cornea which consists of lacrimal fluid and of the secretion of the meibomian and conjunctival glands. It is composed of three layers: (1) the mucin layer (or mucous layer); (2) the lacrimal layer (or aqueous layer) and (3) the oily layer (or lipid layer). Some consider the innermost aqueous and mucin gel layer to be a single layer.

*'unstable tear film'* generally refers to a tear film having a higher than normal relative abundance of cholesterol, especially of cholesterol esters.

*'stabilising a tear film'* or *'improving stability of a tear film'* generally refers to treatment whereby the tear film break up time is increased beyond that time for tear film break up observed in the absence of treatment.

*'tear film break up time'* generally refers to a period from a last blink to the first observance of some instability in tear characteristics. It can be measured by a number of methods including fluorescein staining as in Lee JHL and Kee CW 1988 Kor J. Ophthalmol, 2: 69-71 or by high-speed videokeratoscopy as in Iskander et al. 2005 IEEE Transactions on Biomedical Engineering vo. 52: 1939 -1949, or by the methods described herein.

*'increasing tear film break up time'* or *'improving tear film break up time'* generally refer to lengthening the period from a last blink to the first observance of instability in tear characteristic.

*'ocular surface'* generally refers to corneal and conjunctival tissues and related surfaces that are exposed to tear film.

*'ocular surface inflammatory disorder'* generally refers to acute or chronic inflammation of an ocular surface. The disorder may include acute or chronic inflammation of spatially related tissues and surfaces including epithelia, glands, ciliated structures and related tissues at the margins of conjunctival tissues.

*'ocular surface inflammatory disorder that is characterised by an unstable tear film'* generally refers to a subclass of inflammatory disorders whereby the observed inflammation is either associated with, or caused by an unstable tear film. An unstable tear film may precede the development of the inflammatory disorder, it may develop simultaneously with the development of the inflammatory disorder, or it may proceed the inflammatory disorder.

*'blepharitis'* generally refers to inflammation of the eyelid. Therefore, it is an example of an ocular surface inflammatory disorder.

*'posterior blepharitis'* is an inflammatory disorder of the posterior eyelid, and especially of related conjunctival tissue. Therefore, posterior blepharitis is an example of an ocular surface inflammatory disorder that is characterised by an unstable tear film. Posterior blepharitis is distinguished from 'anterior blepharitis', the latter being an inflammation of the anterior eyelid. In some circumstances, an ocular surface inflammatory disorder that is characterised by an unstable tear film may include clinical or sub clinical manifestations of anterior blepharitis.

*'meibum -producing tissue'* is generally understood as being the tissue consisting of or including the meibomian glands and related ducts. Meibomian glands generally open into the conjunctiva lining in the posterior eyelid.

The words '*treat'* or *'treatment'* generally refer to therapeutic treatment wherein the object is to slow down (lessen) an undesired physiological change or disorder. For purposes of this invention, beneficial or desired clinical results include, but are not limited to, alleviation of symptoms, diminishment of extent of disease, stabilized (i.e., not worsening) state of disease, delay or slowing of disease progression, amelioration or palliation of the disease state, and remission (whether partial or total), whether detectable or undetectable. Treatment may not necessarily result in the complete clearance of a condition but may reduce or minimise complications and side effects of a condition and the progression of infection. The success or otherwise of treatment may be monitored by physical examination of the individual by known ophthalmological techniques.

As used herein, except where the context requires otherwise, the term *"comprise"* and variations of the term, such as *"comprising", "comprises"* and *"comprised',* are not intended to exclude further additives, components, integers or steps.

### B. Methods of treatment

In a clinical study described herein, the inventors treated a range of individuals having one or more manifestations of blepharitis and in particular, posterior blepharitis. The study has demonstrated a significant improvement in tear film stability in each individual.

In particular, the study has demonstrated that the aqueous layer is less important for tear stability, provided that meibum has a constituency of lipid components appropriate for formation of a functional lipid layer, something that was not known at the time of this invention.

The study further demonstrates that none of phospholipids, sphingolipids, wax esters, triglycerides or free fatty acid hydrocarbons are critical for formation of a functional lipid layer, provided that the relative abundance of cholesterol can be regulated. This is novel with respect to earlier studies on the unknown relative importance of cholesterol presence in chronic blepharitis given that relative abundance of cholesterol alone appeared not to be critical in some normal variant patients' meibum [McCulley *supra*]*.* Thus, described herein is a method of stabilising a tear film in an individual having an ocular surface inflammatory disorder including the following steps:
- providing an individual having an ocular surface inflammatory disorder that is characterised by an unstable tear film;
- providing a compound to an ocular surface of the individual to reduce the synthesis of a cholesterol by a meibum -producing tissue;
thereby stabilising the tear film in the individual.

The compound for reducing synthesis of cholesterol by a meibum -producing tissue may be a HMG CoA reductase inhibitor, for example a statin. Statins have been used primarily for reducing serum cholesterol levels. While the current invention has found that the effect of statins on cholesterol synthesis by meibum -producing tissues is similar to that observed in tissues that synthesise serum cholesterol, the outcome of the effect, namely stabilisation of tear film, as compared with reduction of serum cholesterol, is different, and given the different indications, not predictable.

According to the invention, the statin may have a hexahydronaphthalene ring. Examples include lovastatin (US patent no. 4231938), simvastatin (US patent no. 4,444,784) or pravastatin (US patent no. 4,346,227).

Other useful statins include those having a fluorophenyl ring, for example fluvastatin (US patent no. 4,739,073), atorvastatin (US patent no. 4,681,893), rosuvastatin (European patent no. 0521471), pitavastatin (US patent no. 5,856,336), dalvastatin (European Patent Application Publication No. 363934A1), glenvastatin (US patent no. 4,925,852), bervastatin (US patent no. 5,082,859), cerivastatin (US patent no. 5,502,199), or carvastatin.

A particularly preferred statin is atorvastatin.

Atorvastatin may be applied in an amount of 1 to 15 drops per day from a 50µM atorvastatin solution. The application of such a solution may be for 1 to 4 weeks or longer.

In one specific embodiment, especially where the ocular surface inflammatory disorder is posterior blepharitis, the compound for reducing synthesis of cholesterol by a meibum -producing tissue may be an HMG CoA reductase inhibitor, preferably a statin, more preferably Atorvastatin.

According to the disclosure, especially where the ocular surface inflammatory disorder is posterior blepharitis, the compound for reducing synthesis of cholesterol by a meibum -producing tissue may be an androgen. Examples of suitable androgens include estrogens, including estrogen esters, 17- beta estradiol.

A further unexpected advantage of statin administration to the ocular surface is the reduction in inflammation of ocular surfaces mediated by anti-inflammatory properties of statins on conjunctival, corneal and related surfaces. Thus while statin therapy described herein is focussed on, and attends primarily to correction of tear film instability, the therapy also provides for improvements in inflammation. This approach is distinguished from other chemotherapies that have largely focussed on anti-inflammatory or immunosuppressive therapies using steroids, cyclosporins or non statins in the form of statin like structures that are devoid of a lactone ring or heptanoic acid groups in protected or unprotected form that are required for inhibition of cholesterol synthesis. The latter chemotherapy is focussed on PDE6 -related conditions or inflammatory disorders with no suggestion of cholesterol metabolism dysfunction (see US2004/0248972A1 to Ambit Biosciences Corp).

The statin may be administered with an anti-inflammatory agent in the form of a steroid. Examples include prednisolone, fluorometholone, triamcinolone, rimexolone, betamethasone. Importantly, the inventors have found that the combination of atorvastatin with steroid for treatment of ocular inflammation establishes an outcome that is greater than the sum of outcomes of treatment with atorvastatin and steroid alone. Therefore, a new synergistic relationship has been identified by the inventors that arises from the combination of statin and steroid therapy, and especially from atorvastatin and steroid therapy.

Thus, the method may include the further step of:
- providing an anti -inflammatory compound and/or an immunosuppressant to an ocular surface of the individual.

According to the invention, the compound for reducing synthesis of cholesterol by a meibum -producing tissue may be a HMG CoA reductase inhibitor. According to the disclosure, the compound for reducing synthesis of cholesterol by a meibum -producing tissue may be other than a HMG CoA reductase inhibitor. Regarding the latter, for example, the compound may be a hormone or androgen. Given that these androgen compounds generally do not have anti-inflammatory properties, the inventors have recognised that where a hormone is used to correct, alter or modify cholesterol synthesis by meibum-producing tissue, an anti-inflammatory compound and/or an immunosuppressant is generally required for treatment or at least amelioration of those ocular surface inflammatory disorders that are characterised by tear film instability, such as posterior blepharitis.

Therefore, the disclosure provides a method of stabilising a tear film in an individual having an ocular surface inflammatory disorder including the following steps:
- providing an individual having an ocular surface inflammatory disorder that is characterised by an unstable tear film;
- providing a compound in the form of a hormone or androgen to an ocular surface of the individual to reduce the synthesis of a cholesterol by a meibum -producing tissue;
- providing an anti -inflammatory compound and/or an immunosuppressant to an ocular surface of the individual;
thereby stabilising the tear film in the individual.

The anti -inflammatory compound may be a steroid as described herein. The immunosuppressant may be a cyclosporin.

The hormone (or androgen) and anti-inflammatory (or immunosuppressant) may be provided in the form of a single dosage unit, whereby both compounds are provided in the one formulation, enabling administration from a single formulation. Otherwise, these compounds may be formulated separately in which case they can be administered sequentially or simultaneously.

The statin may be provided together with a hormone or androgen. For example, atorvastatin may be provided with an estrogen ester. Advantageously, in this embodiment the statin can be added directly to a formulation adapted for ophthalmic use, such as an eye drop. For example, atorvastatin could be added to iDestrin (17-beta -estradiol; *Nascent Pharmaceuticals)* or to Androgen Tears *(Allergan)* in the amounts described herein.

The statin may be provided together with a tear lubricant. In one example the tear lubricant is a liquid formulation, with particular examples including Cystane (*Alcon Laboratories*)*,* Optive *(Allergan),* Liquid Film Tears, Cellufresh *(Allergan)* and Visine. In another example, the tear lubricant is a gel, with particular examples including Systane Ultra *(Alcon Laboratories),* Polyvisc *(Alcon Laboratories),* Celluvisc *(Allergan).* In another example the tear lubricant is an ointment, with particular examples including Lacrilube (*Allergan*) and Gel tears.

The statin may be provided together with another ocular therapeutic agent, for example a glaucoma eye drop formulation. These agents may be useful for reducing intraocular pressure. Examples include Combigan *(Allergan),* Duotrar (*Alcon Laboratories),* Ganfort (*Allergan*), Azarga (*Alcon Laboratories),* and Salacon.

The above described methods may include the further step of
- providing an antibiotic to an ocular surface of the individual.

The antibiotic may be bacteriostatic or bacteriocidal. Typically it is effective against *Staphylococcus aureus* and other bacteria commonly found in ocular surface disorders, especially those bacteria that invade at or are near meibum -producing tissue.

The methods described herein are particularly useful for the treatment of posterior blepharitis and other related conditions.

In one embodiment the condition may be selected from the group consisting of Sjogren's syndrome, Allergic (kerato)conjunctivitis eg Atopic keratoconjunctivitis, Rosacea, Ocular cicatricial pemphigoid, Inflamed pterygium or pingueculum, Marginal keratitis, Phlyctenulosis, Post-operative inflammatory states eg Post-refractive surgery, Thygeson's superficial punctate keratitis, Superior limbic keratoconjunctivitis, Episcleritis and lipid keratopathy.

The methods are also particularly useful for minimising one or more symptoms or clinical or sub clinical manifestations of an ocular surface disorder characterised by tear film instability including corneal epithelial dryness, conjunctival redness and the like.

Where the method is for the treatment of posterior blepharitis, the person provided for or selected for may or may not have symptoms of dry eye disease.

### C. Compositions

The disclosure also relates to compositions for use according to the invention, particularly compositions adapted for ophthalmic administration, including solutions and suspensions, topical ointments and inserts, examples of which are described below.

There is provided a composition formulated for ophthalmic administration including a compound for reducing the synthesis of a cholesterol by a meibum -producing tissue. The composition may be adapted to enable execution of anyone of the above described methods.

The compound for reducing the synthesis of a cholesterol by a meibum -producing tissue (herein the therapeutic compound) may be an inhibitor of
HMG CoA reductase, especially a statin, such as atorvastatin. Particularly preferred are solutions of atorvastatin for ophthalmic administration having from about 1 to 500µM atorvastatin and excipients, diluents etc as generally used in the ophthalmic arts.

The therapeutic compound may be an androgen.

A composition for use according to the invention may further include an anti-inflammatory and/or immunosuppressive agent.

### C.1 Ophthalmic solutions and suspensions

The compositions described herein may further comprise pharmaceutically acceptable excipients conventional to the pharmaceutical art. Typical of such pharmaceutically acceptable excipients include osmotic/tonicity-adjusting agents, preservatives, one or more pharmaceutically acceptable buffering agents and pH-adjusting agents, solubilizing agents, vehicles and other agents conventional in art that may be used in formulating an ophthalmic composition.

The ophthalmic compositions are required to be isoosmolar with respect to the ophthalmic fluids present in the human eye. These solutions are characterized by osmolalities of 250-375 mOsm/kg. Osmolality of the solutions is adjusted by addition of an osmotic/tonicity adjusting agent. Osmotic agents that may be used in the compositions for use according to the present invention to make it isotonic with respect to the ophthalmic fluids present in the human eye, are selected from the group comprising sodium chloride, potassium chloride, calcium chloride, sodium bromide, mannitol, glycerol, sorbitol, propylene glycol, dextrose, sucrose, and the like, and mixtures thereof.

Further, the ophthalmic compositions for use according to the present invention may comprise preservatives in effective amounts. Antimicrobial effective amounts of a preservative may be determined by performing preservative efficacy tests or antimicrobial effectiveness tests. These tests are inter alia described in chapter 51 of the United States Pharmacopoeia 29-National Formulary 24 (USP 29-NF 24). The preservatives may be used in an amount within the concentration ranges described in standard
reference books like 'Remington's Pharmaceutical Sciences' and 'Handbook of Pharmaceutical Excipients'.

The preservative may be selected from: Quaternary ammonium compounds such as benzalkonium chloride (BKC) and benzethonium chloride; Organic mercurials such as phenylmercuric acetate, phenylmercuric nitrate and thimerosal; Parabens such as methyl and propyl paraben; ethyl paraoxybenzoate or butyl paraoxybenzoate; Acids and their pharmaceutically acceptable salts such as sorbic acid, potassium sorbate, boric acid, borax, salicylic acid; Substituted alcohols and phenols such as chlorobutanol, benzyl alcohol; phenyl ethanol; Amides such as acetamide; and the like, and combinations thereof.

The ophthalmic composition may be self preserving. The ingredients that make the composition self preserving include, but are not limited to, combination of zinc salts and boric acid in presence of tromethamine.

In order to achieve, and subsequently maintain, an optimum pH, the ophthalmic compositions essentially contain a pH adjusting agent and/or a buffering agent. The preferred range of pH for an ophthalmic formulation is about 6.8 to about 7.8, and the most preferred pH is about 7.4.

The ophthalmic compositions for use according to the present invention comprise a pharmaceutically acceptable pH adjusting agent that may be selected from the group comprising acetic acid or salts thereof, boric acid or salts thereof, phosphoric acid or salts thereof; citric acid or salts thereof, tartaric acid or salts thereof, sodium hydroxide, potassium hydroxide, sodium carbonate, sodium hydrogencarbonate, trometamol, and the like and mixtures thereof. Particularly, preferred pH adjusting agents that may be used in the composition include acetic acid, hydrochloric acid, sodium carbonate and sodium hydroxide. These agents are used in amounts necessary to produce a pH ranging from about 6.0 to about 8.0.

Besides above mentioned ingredients, the formulation of the invention may include a number of additional components to provide various effects, as is well known in this field. For example, the composition may include edetate disodium, which may function as a co-preservative and chelating agent.

### C.2 Ophthalmic ointments

A composition of the invention may be provided for use in a method of treatment described herein in the form of an ophthalmic ointment. An ophthalmic ointment is generally a viscous, homogeneous mixture comprising the therapeutic compound and relatively high concentrations of solid, water-soluble polymers (described hereinafter) dissolved in an aqueous medium.

The concentration of the therapeutic compound in the novel formulation can vary over a wide range depending on the solid water-soluble polymers of choice, the disease to be treated, the clinically observed rate of elution, and other considerations. In general, at least a therapeutically effective amount is used which amount is within established concentration ranges of safety and efficacy. Thus, the novel ointments can contain the therapeutic compound in varying concentrations, e.g., from 0.1 weight percent to its maximum solubility in an aqueous medium. Depending on the factors illustrated above, the concentration of other medicaments would vary within established levels of safety, efficacy, and effectiveness.

The polymers of the ointment are normally-solid materials which have well-defined average molecular weights ranging from about 15,000 g/mol to about 500,000 g/mol. The polymers may be water-soluble in acidic and basic media, e.g., pH of from 4 to about 8.

An amount of solid, compatible, non-toxic, water-soluble polymer is employed in the novel ointment which is generally sufficient to impart to such ointment a slow-flow characteristic which is substantially shape-retaining as it (ointment) is forced from the opening of a tube (such as a smaller version of an ordinary tube of toothpaste) under mild hand squeezing of the tube. Such preferred ointments are soft and easily deformed under mild pressure, as by the pressure resulting from a blink of the eye. The viscosity of the novel ointment, as determined by Brookfield Viscomiter LVT, Spindle No. 3, Speed 12 rpm, at 25° C., can vary over a wide range, e.g., from a few hundred centipoises and upwards to 50,000, and even higher. In general, a maximum viscosity of about 25,000 centipoises should be sufficient for most purposes. A lower viscosity limit which is suitable is about 2000 centipoises. The concentration of the solid, water-soluble polymer component can be up to about 35 weight percent, and even higher, based on the total weight of the ointment. In general, a sufficient amount of polymer is employed to result in an ointment having the desired viscosity, for example, an ointment containing from 12 to 30 weight percent polymer.

The novel ointments, as indicated previously, are viscous, homogeneous formulations comprising water, polymer, and therapeutic compound. They generally are not "greasy" like petrolatum. They are non-toxic and non-irritating to the cornea and mucosa tissue. The novel ointments may initially act after the first blink similarly to a plano-hydrogel lens. Upon tear dilution the ointment may increasingly resemble tears.

The polymers that may be suitable include the solid-water-soluble acrylic polymers, aliphatic amide polymers, and ethyleneimine polymers. Highly desirable polymers are those which contain a sufficient amount of a water-solubilizing comonomer chemically combined therein in order to impart to the polymer a water-solubility characteristic.

### C.3 Ophthalmic inserts

The composition for use according to the invention may be provided in the form of an ophthalmic insert. The insert may be a non-toxic solid water soluble polymer based material that is adapted to be placed into the cul-de-sac of the eye to obtain long term release of the composition of the invention.

The polymer used to form the inserts may be any water soluble non-toxic polymer. For example, one may employ water soluble polymers of cellulose derivatives such as methylcellulose, alkali carboxyloweralkyl cellulose (sodium carboxymethyl cellulose), hydroxyloweralkyl cellulose, (hydroxmethyl cellulose,
hydroxyethyl cellulose, hydroxypropyl cellulose), hydroxyloweralkyl-lower alkyl cellulose, (hydroxypropylmethyl cellulose); (Lower alkyl meaning from 1-4 carbon atoms); natural products (natural gums), such as gelatin, metal alginates (Na, K, Ca, Zn, Al), pectins, tragacanth, karaya, chondrus, agar, acacia; the starch derivatives such as hydroxyethyl starch ethers, hydroxypropyl starch; dextran, lower hydroxyalkyl dextran, carboxyloweralkyl dextran, polyalkylene glycols (polyethylene and polypropylene glycols), as well as other synthetic derivatives such as poly vinylmethyl ether, poly ethylene oxide, neutralized carbopol and xanthan gum, and mixtures of said polymer.

The molecular weight of these polymers useful for the purposes described herein may be at least 30,000 g/mol to about 1,000,000 g/mol or more although it is clear that for the purpose of this invention the molecular weight of the polymer is not critical. Water soluble polymers can be used having an average molecular weight which will afford dissolution of the polymer in any desired length of time. The inserts, therefore, can be prepared to allow for retention and accordingly effectiveness in the eye for any desired period.

The insert can be of any desired shape and size which in its outer limits can cover the entire globe of the eye including the cornea and sclera and extend from the inferior, superior, nasal and temporal limits to the conjunctival sac. Accordingly the insert can be in the form of a square, rectangle, oval, circle, doughnut, semicircle, 1/4 moon shape, and the like. Preferably the insert is in the form of a rod, doughnut, oval or 1/4 moon.

The insert can be readily prepared, for example, by dissolving the polymer in a suitable solvent and the solution evaporated to afford a thin film of the polymer which can then be subdivided to prepare inserts of appropriate size. Alternatively the insert can be prepared by warming the polymer and then molded to form a thin film. Preferably, the inserts are prepared by molding or extrusion procedures well known in the art. The molded or extruded product can then be subdivided to afford inserts of suitable size for administration in the eye.

In order to properly fit into the cul-de-sac of the eye without excessive irritation, and yet to be effective for its intended use, the insert should have a surface area of from about 5 to about 800 sq. mm., preferably 5-400 sq. mm., and especially 5-100 sq. mm., and most especially 5-50 sq. mm.; a length of from about 1-30 mm., preferably about 5-20 mm., and especially 2-15 mm.; and a width and height of from about 0.25 mm. to about 30 mm., preferably from about 1-10 mm., and especially 1-5 mm.

It is preferred that the ophthalmic inserts of the present invention be formed so that they are smooth and do not have any sharp edges or corners which could cause damage to the eye.

The ocular inserts can also contain plasticizers, buffering agents and preservatives. Compositions for use according to the invention may thus contain these materials along with the water soluble polymer. Plasticizers suitable for this purpose must, of course, also be completely soluble in the lacrimal fluids of the eye. Examples of suitable plasticizers that might be mentioned are water, polyethylene glycol, propylene glycol, glycerine, trimethylol propane, di and tripropylene glycol, hydroxypropyl sucrose and the like. Typically, such plasticizers can be present in the ophthalmic insert in an amount ranging from up to 1 about 40% by weight.

Suitable water soluble preservatives which may be employed in the insert are sodium bisulfite, sodium thiosulfate, ascorbate, benzalkonium chloride, chlorobutanol, thimerosal, phenylmercuric acetate, phenylmercuric borate, parabens, benzyl alcohol and phenylethanol. These agents may be present in amounts of from 0.001 to 5% by weight of solid insert, and preferably 0.1 to 2%.

Suitable water soluble buffering agents are alkali, alkali earth carbonates, phosphates, bicarbonates, citrates, borates, and the like, such as sodium phosphate, citrate, borate, acetate, bicarbonate and carbonate. These agents may be present in amounts sufficient to obtain a pH of the system of between 5.5 to 8.0 and especially 7-8; usually up to about 2% by weight of polymer.

The water soluble polymer inserts when applied to the eye of a patient having dry eye syndrome with at least one symptom of corneal pathology, photophobia, burning, foreign body sensations and instantaneous or near instantaneous break up time measurements, significantly improves patient comfort for at least several hours, prolongs the break up time and thickens the pre-corneal tear film for at least several hours compared to little or no improvement with commercially available liquid artificial tears.

The ophthalmic insert may contain any convenient weight of polymers. As a practical matter, too little polymer will lead to dissolution over too short a period of time while too much may make the insert too bulky. Accordingly, it is preferred that the insert contain from about 1 mg. to 1000 mg. of water soluble polymer, more particularly from 2 to 300 mg., and especially from 5 to 100 mg. The time of dissolution of the insert is dependent upon each patient's natural supply of tears as well as the particular polymer employed. Usually the time of dissolution is between about 4 hours to 7 days, and preferably 6 to 24 hours, but suitably may be as little as one hour.

### Examples

### Example 1

### Abstract

Aim: To investigate topical statin as a novel therapy for dry eyes associated with blepharitis. Methods: 10 patients with symptoms and signs of dry eye and blepharitis were enrolled in a prospective pilot study of topical statin therapy over 1 month. The primary outcome measure was corneal fluorescein staining. Results: An improvement in corneal fluorescein staining by ≥ 3 points from baseline to completion of the trial at week four was found in 6 of 10 patients in the study eye. Subjective dry eye symptoms improved (p = 0.005). Topical statin significantly improved the blepharitis score (p = 0.011) and the tear film break-up time (p=0.005). There were no serious or irreversible side-effects. Conclusions: Topical statins are a suitable therapy for patients with dry eye and blepharitis. They can be easily manufactured and could be widely available.

### Aim:

To study the potential for topical statins in reducing the symptoms and signs of dry eyes associated with blepharitis

### Study Design:

A non-comparative pilot study was conducted to demonstrate the efficacy and safety of topical statin therapy and to power a planned prospective randomized control trial. 10 patients were enrolled and completed the study.

Prior to this pilot study, topical statin was assessed *in vitro* using human primary corneal epithelial cell cultures established from donor corneal tissue (Lions NSW Eye Bank, Sydney, Australia) and corneal and conjunctival cell-lines.

Patient tear samples were collected at enrolment and study completion (4 weeks) and stored at -80°C. Multiplex assays to determine tear levels of IL-1β, IL-2, IL-4, IL-5, IL-6, IL-10, IL-12, IL-13, TNFα, IFNγ, MCP-1, & RANTES were conducted.

### Inclusion criteria:

Patients with
1) Any symptoms of dry eyes and blepharitis
2) Any signs of dry eyes:
   BUT (tear break up time) ≤ 5 sec, or
   Punctate epithelial erosions in at least one eye
3) Any signs of blepharitis:
   Anterior lid margin - grease, skin scales, collarettes, frank ulcers, loss of lashes and inflammation
   Posterior lid margin - cheesy secretions, blocked meibomian glands >1/3, telangiectasia, meibomitis, notched lid margin and chalazia
4) Male or female patients greater or equal to 18 years of age
5) Patients able to give written informed consent and comply with study protocol

### Exclusion criteria:

1) Patients who have received an experimental drug within the past 6 weeks
2) Patients on any topical treatment other than artificial tears 4 weeks prior
3) Patients who wear contact lenses
4) Active immunological melts - Rheumatoid arthritis, Systemic lupus erythematosus, Polyarteritis nodosa, Microscopic polyangiitis, Wegeners, Mooren's ulcer
6) Active bacterial, fungal or amoebic infective ulcers
7) Acute Herpes simplex virus, Herpes zoster virus or other keratitis or inflammatory conjunctival disease
8) Vitamin A deficiency
9) Recurrent corneal erosion syndrome
10) Pregnant or lactating females

### Drug:

Atorvastatin (50 microM) one drop 8 times a day to both eyes

### Efficacy Evaluation:

1) Subjective
   Total symptoms score
   Face score (Goto *et al* 2002)
2) Objective
   a) Tear film break-up time (BUT)
   b) Corneal fluorescein staining
   b) Blepharitis score
   c) Bulbar conjunctival injection
   d) Concomitant use of topical therapy

### Safety evaluation:

1) BSCVA
2) Cataract
3) IOP
4) Adverse reactions

### Statistical analysis:

Prior to analysis, information from the completed study proformas was entered into a Microsoft Access database designed for the study. For efficacy variables, only data from the worst eye was analysed. The worst eye was defined as the eye at enrolment with the highest sum of the global symptom, corneal fluorescein staining and blepharitis scores. If both eyes were affected to the same degree the right eye was selected for analysis. Data from both eyes was included for all safety analyses.

Descriptive statistics were used to summarise all continuous and categorical variables. Patients were evaluated at weeks 1, 2, 3 and 4. However, the primary endpoint was at trial completion (4 weeks). A p value < 0.050 was considered statistically significant. The statistical software used was SPSS version 13 (SPSS Inc., Chicago, Illinois, USA).

For parametric data, differences with respect to change from baseline were assessed with the paired t-test. Differences within each group, with respect to change from baseline in non-parametric data, were assessed with the Wilcoxon signed ranks test.

### Results:

Enrolled prospectively in the pilot study were 10 eyes of 7 female and 3 male patients with dry eye and blepharitis. Their mean age was 70 years (range 53 to 84 years). All patients completed the study (there were no losses to follow-up and no withdrawals). There were no patients with dry eye associated with primary or secondary Sjögren's syndrome. No patient had glaucoma and 3 had had prior intraocular surgery in the study eye.

Prior to the study, 6 patients were using topical lubricants an average of 6 times a day (range 2 to 8, SD 2.3); 4 of these patients were using preservative-free lubricants. At study completion, 2 patients had ceased their topical lubrication, and 3 of the remaining 4 patients had reduced the frequency of lubricant use to an average of 4 times a day (range 3 to 6, SD 0.6). Two patients were using lubricating ointment once a day, this did not change during the study. One patient had had punctal occlusion in the study eye. 4 patients were performing lid massage with hot compresses and 4 were using a cotton bud for lid hygiene, 1 with bicarbonate of soda solution and 2 with shampoo. Patients were asked not to change these regimes during the study period.

Patients were instructed to use statin eyedrops 8 times a day. 7 patients were fully compliant. 2 patients used them 7 times a day and one patient only 5 times a day during the study.

### Primary outcome:

The corneal fluorescein staining score improved in 9 of the 10 patients by more than one point in the study eye (p = 0.007). An improvement in corneal fluorescein staining by three or more points from baseline to completion of the trial at week four was found in 6 of 10 patients in the study eye.

**Table 1: Summary of patient demographics and results in the topical statin study.**

| **Patient** | **Time** | **1** | **2** | **3** | **4** | **5** | **6** | **7** | **8** | **9** | **10** |
|---|---|---|---|---|---|---|---|---|---|---|---|
| Eye | | R | R | R | R | R | L | R | L | R | R |
| Age (years) | | 54 | 79 | 59 | 69 | 53 | 80 | 70 | 84 | 67 | 84 |
| Sex | | F | F | F | M | F | F | F | F | M | M |
| Tear film break-up time (seconds) | | 1 | 1 | 3 | 2 | 1 | 4 | 4 | 6 | 4 | 5 |
| Schirmers 1 test result (mm) | | 27 | 9 | 0 | 9 | 7 | 11 | 33 | 8 | 7 | 28 |
| Corneal fluorescein score¹ | Baselin e | 0 | 6 | 5 | 3 | 9 | 3 | 4 | 3 | 3 | 7 |
| | 1 month | 0 | 2 | 2 | 0 | 4 | 0 | 2 | 1 | 1 | 0 |
| Blepharitis score | Baselin e | 7 | 7 | 3 | 5 | 8 | 8 | 7 | 5 | 9 | 10 |
| | 1 month | 4 | 7 | 2 | 5 | 2 | 5 | 4 | 4 | 7 | 5 |
| Global Symptom score | Baselin e | 11 | 11 | 7 | 4 | 14 | 7 | 13 | 8 | 13 | 7 |
| | 1 month | 0 | 3 | 4 | 1 | 2 | 0 | 0 | 0 | 5 | 0 |
| Face scale score² | Baselin e | 4 | 5 | 7 | 4 | 5 | 5 | 9 | 4 | 6 | 5 |
| | 1 month | 3 | 5 | 5 | 3 | 4 | 4 | 5 | 2 | 4 | 1 |

| | | | | | | | | | | | |
|---|---|---|---|---|---|---|---|---|---|---|---|
| 1 = National Eye Institute fluorescein scoring system (0-15) (Lemp, 1995), 2= Facial analogue scale (Goto et al, 2002, R = right, L = left, M = male, F = female | | | | | | | | | | | |

### Secondary outcomes:

The tear film break-up time (BUT) improved significantly in all 10 patients (p=0.005, Table 2). In 8 patients the blepharitis score improved and in 2 there was no change (p = 0.011). Conjunctival injection reduced in 5 patients and remained the same in 5 (p = 0.025). There was no significant change in the Schirmer I test result.

The global score for subjective symptoms of dry eye improved significantly in all 10 patients (p = 0.005) (see Table 3). For individual symptoms scores, there was significant improvement from enrolment to week four for dryness, foreign body sensation, discomfort, redness, watering, and face score. There was no significant change from enrolment to week four for blurred vision or photophobia.

**Table 2: Change in signs in the study eye from baseline to completion of the dry eye pilot study at week four.**

| **Sign** | **Improved (n)** | **No change (n)** | **Worse (n)** | **p value** |
|---|---|---|---|---|
| Corneal fluorescein score | 9 | 1 | 0 | 0.007* |
| Tear film BUT | 10 | 0 | 0 | 0.005* |
| Blepharitis score | 8 | 2 | 0 | 0.011* |
| Schirmer I test | 5 | 0 | 5 | 0.759 |
| Conjunctival injection | 5 | 5 | 0 | 0.025* |

| | | | | |
|---|---|---|---|---|
| BUT = break-up time, SE = study eye, FE = fellow eye and n = number of patients. | | | | |

**Table 3: Change in symptom score in the study eye from baseline to completion of the dry eye pilot study at week four.**

| **Symptom** | **Improved** | **No change (n)** | **Worse** | **p value** |
|---|---|---|---|---|
| Global symptom score | 10 | 0 | 0 | 0.005* |
| Dryness | 8 | 1 | 1 | 0.029* |
| Foreign body sensation | 10 | 0 | 0 | 0.004* |
| Blurred vision | 3 | 7 | 0 | 0.109 |
| Discomfort | 9 | 0 | 1 | 0.008* |
| Photophobia | 5 | 4 | 1 | 0.084 |
| Redness | 5 | 5 | 0 | 0.046* |
| Watering | 7 | 3 | 0 | 0.015* |
| Face score | 9 | 1 | 0 | 0.07* |

| | | | | |
|---|---|---|---|---|
| n = number of patients. | | | | |

### Safety data

From baseline to study completion, safety data analysis showed no significant change in the study or fellow eye for BCVA (Table 4) and no patient developed cataract. There was no change in IOP in the study eye. In the fellow eye the IOP was lower at trial completion in 8 of 10 patients (p = 0.035)

No adverse events were reported and 9 of 10 patients rated the acceptability and ease of use of topical statin therapy as 'very good'. One patient gave a rating of 'neither good nor bad'.

**Table 4: Change in signs in the study eye from baseline to completion of the dry eye pilot study at week four.**

| **Sign** | **Improved** | **No change (n)** | **Worse (n)** | p value |
|---|---|---|---|---|
| BCVA SE | 6 | 3 | 1 | 0.058 |
| BCVA FE | 4 | 6 | 0 | 0.063 |
| Intraocular pressure SE | 5 | 3 | 2 | 0.127 |
| Intraocular pressure FE | 8 | 1 | 1 | 0.035* |

| | | | | |
|---|---|---|---|---|
| BCVA = best-corrected visual acuity, BUT = break-up time, SE = study eye, FE = fellow eye and n = number of patients. | | | | |

### References:

Blanco-Colio LM, Tunon J, Martin-Ventura JL, Egido J. Anti-inflammatory and immunomodulatory effects of statins. Kidney Int. 2003 Jan;63(1):12-23.
Goto E, Monden Y, Takano Y, Mori A, Shimmura S, Shimazaki J, Tsubota K. Treatment of non-inflamed obstructive meibomian gland dysfunction by an infrared warm compression device. Br J Ophthalmol. 2002 Dec;86(12):1403-7.
Lemp MA. Report of the National Eye Institute/Industry Workshop on clinical trials in dry eyes. CLAO J 1995;21(4):221-32.
Schiffman RM, Christianson MD, Jacobsen G, Hirsch JD, Reis BL. Reliability and validity of the Ocular Surface Disease Index. Arch Ophthalmol. 2000 May;118(5):615-21.

## Claims

1. A HMG CoA reductase inhibitor for use in a method of treating an ocular surface inflammatory disorder in an individual:
wherein the ocular surface inflammatory disorder is **characterised by** an unstable tear film;
wherein the HMG CoA reductase inhibitor is administered to the ocular surface of the individual;
wherein the HMG CoA reductase inhibitor reduces the synthesis of a cholesterol by a meibum-producing tissue thereby stabilising the tear film in the individual; and
wherein the HMG CoA reductase inhibitor is a statin.

2. The HMG CoA reductase inhibitor for use according to claim 1 wherein the statin has a hexahydronaphthalene ring or a fluorophenyl ring.

3. The HMG CoA reductase inhibitor for use according to claim 2 wherein the statin is selected from lovastatin, simvastatin, pravastatin, fluvastatin, atorvastatin, rosuvastatin, pitavastatin, dalvastatin, glenvastatin, bervastatin, cerivastatin, or carvastatin.

4. The HMG CoA reductase inhibitor for use according to claim 3 wherein the statin is atorvastatin.

5. The HMG CoA reductase inhibitor for use according to claim 4, wherein atorvastatin is administered in an amount of 1 to 15 drops per day from a 50µM atorvastatin solution.

6. The HMG CoA reductase inhibitor for use according to claim 5 wherein the administration is for 1 to 4 weeks.

7. The HMG CoA reductase inhibitor for use according to any one of claims 1 to 6, wherein the HMG CoA reductase inhibitor further comprises an anti-inflammatory compound and/or an immunosuppressant.

8. The HMG CoA reductase inhibitor for use according to any one of claims 1 to 6, wherein the HMG CoA reductase inhibitor is separately administered with an anti-inflammatory compound and/or an immunosuppressant.

9. The HMG CoA reductase inhibitor for use according to claim 7 or 8 wherein the anti-inflammatory compound is a steroid.

10. The HMG CoA reductase inhibitor for use according to any one of claims 7 to 9 wherein the immunosuppressant is a cyclosporin.

11. The HMG CoA reductase inhibitor for use according to any one of claims 1 to 10 wherein the HMG CoA reductase inhibitor further comprises an antibiotic.

12. The HMG CoA reductase inhibitor for use according to any one of claims 1 to 11 wherein the disorder is posterior blepharitis.

13. A composition formulated for ophthalmic administration, comprising a HMG CoA reductase inhibitor and further comprising an anti-inflammatory compound and/or an immunosuppressant, wherein the HMG CoA reductase inhibitor is a statin, optionally wherein the HMG CoA reductase inhibitor is atorvastatin.

## Patentansprüche

1. HMG-CoA-Reduktase-Inhibitor zur Verwendung in einem Verfahren zur Behandlung einer entzündlichen Augenoberflächenerkrankung bei einem Individuum:
wobei die entzündliche Augenoberflächenerkrankung durch einen instabilen Tränenfilm gekennzeichnet ist;
wobei der HMG-CoA-Reduktase-Inhibitor an die Augenoberfläche des Individuums verabreicht wird;
wobei der HMG-CoA-Reduktase-Inhibitor die Synthese eines Cholesterins durch ein Meibum produzierendes Gewebe reduziert, wobei auf diese Weise der Tränenfilm bei dem Individuum stabilisiert wird; und
wobei der HMG-CoA-Reduktase-Inhibitor ein Statin ist.

2. HMG-CoA-Reduktase-Inhibitor zur Verwendung nach Anspruch 1, wobei das Statin einen Hexahydronaphthalinring oder einen Fluorphenylring aufweist.

3. HMG-CoA-Reduktase-Inhibitor zur Verwendung nach Anspruch 2, wobei das Statin aus Lovastatin, Simvastatin, Pravastatin, Fluvastatin, Atorvastatin, Rosuvastatin, Pitavastatin, Dalvastatin, Glenvastatin, Bervastatin, Cerivastatin oder Carvastatin ausgewählt ist.

4. HMG-CoA-Reduktase-Inhibitor zur Verwendung nach Anspruch 3, wobei das Statin Atorvastatin ist.

5. HMG-CoA-Reduktase-Inhibitor zur Verwendung nach Anspruch 4, wobei Atorvastatin in einer Menge von 1 bis 15 Tropfen pro Tag aus einer 50 µM Atorvastatin-Lösung verabreicht wird.

6. HMG-CoA-Reduktase-Inhibitor zur Verwendung nach Anspruch 5, wobei die Verabreichung für 1 Woche bis 4 Wochen vorgesehen ist.

7. HMG-CoA-Reduktase-Inhibitor zur Verwendung nach einem der Ansprüche 1 bis 6, wobei der HMG-CoA-Reduktase-Inhibitor ferner eine entzündungshemmende Verbindung und/oder ein Immunsuppressivum umfasst.

8. HMG-CoA-Reduktase-Inhibitor zur Verwendung nach einem der Ansprüche 1 bis 6, wobei der HMG-CoA-Reduktase-Inhibitor mit einer entzündungshemmenden Verbindung und/oder einem Immunsuppressivum getrennt verabreicht wird.

9. HMG-CoA-Reduktase-Inhibitor zur Verwendung nach Anspruch 7 oder 8, wobei die entzündungshemmende Verbindung ein Steroid ist.

10. HMG-CoA-Reduktase-Inhibitor zur Verwendung nach einem der Ansprüche 7 bis 9, wobei das Immunsuppressivum ein Cyclosporin ist.

11. HMG-CoA-Reduktase-Inhibitor zur Verwendung nach einem der Ansprüche 1 bis 10, wobei der HMG-CoA-Reduktase-Inhibitor ferner ein Antibiotikum umfasst.

12. HMG-CoA-Reduktase-Inhibitor zur Verwendung nach einem der Ansprüche 1 bis 11, wobei die Erkrankung eine posteriore Blepharitis ist.

13. Zusammensetzung, formuliert zur ophthalmischen Verabreichung, die einen HMG-CoA-Reduktase-Inhibitor umfasst und ferner eine entzündungshemmende Verbindung und/oder ein Immunsuppressivum umfasst, wobei der HMG-CoA-Reduktase-Inhibitor ein Statin ist, gegebenenfalls wobei der HMG-CoA-Reduktase-Inhibitor Atorvastatin ist.

## Revendications

1. Inhibiteur de la HMG CoA réductase destiné à une utilisation dans une méthode de traitement d'une affection inflammatoire de la surface oculaire chez un sujet,
l'affection inflammatoire de la surface oculaire **se caractérisant par** un film lacrymal instable ;
l'inhibiteur de la HMG CoA réductase étant administré sur la surface oculaire du sujet ;
l'inhibiteur de la HMG CoA réductase diminuant la synthèse d'un cholestérol par un tissu produisant du meibum, en stabilisant ainsi le film lacrymal chez le sujet ; et
l'inhibiteur de la HMG CoA réductase étant une statine.

2. Inhibiteur de la HMG CoA réductase destiné à une utilisation selon la revendication 1, dans lequel la statine comporte un cycle hexahydronaphtalène ou un cycle fluorophényle.

3. Inhibiteur de la HMG CoA réductase destiné à une utilisation selon la revendication 2, dans lequel la statine est sélectionnée parmi la lovastatine, la simvastatine, la pravastatine, la fluvastatine, l'atorvastatine, la rosuvastatine, la pitavastatine, la dalvastatine, la glenvastatine, la bervastatine, la cérivastatine, ou la carvastatine.

4. Inhibiteur de la HMG CoA réductase destiné à une utilisation selon la revendication 3, dans lequel la statine est l'atorvastatine.

5. Inhibiteur de la HMG CoA réductase destiné à une utilisation selon la revendication 4, dans lequel l'atorvastatine est administrée dans une quantité de 1 à 15 gouttes par jour à partir d'une solution à 50 µM d'atorvastatine.

6. Inhibiteur de la HMG CoA réductase destiné à une utilisation selon la revendication 5, dans lequel l'administration a lieu sur une période de 1 à 4 semaines.

7. Inhibiteur de la HMG CoA réductase destiné à une utilisation selon l'une quelconque des revendications 1 à 6, l'inhibiteur de la HMG CoA réductase comprenant en outre un composé anti-inflammatoire et/ou un immunosuppresseur.

8. Inhibiteur de la HMG CoA réductase destiné à une utilisation selon l'une quelconque des revendications 1 à 6, l'inhibiteur de la HMG CoA réductase étant administré séparément avec un composé anti-inflammatoire et/ou un immunosuppresseur.

9. Inhibiteur de la HMG CoA réductase destiné à une utilisation selon la revendication 7 ou 8, dans lequel le composé anti-inflammatoire est un stéroïde.

10. Inhibiteur de la HMG CoA réductase destiné à une utilisation selon l'une quelconque des revendications 7 à 9, dans lequel l'immunosuppresseur est une cyclosporine.

11. Inhibiteur de la HMG CoA réductase destiné à une utilisation selon l'une quelconque des revendications 1 à 10, dans lequel l'inhibiteur de la HMG-CoA réductase comprend en outre un antibiotique.

12. Inhibiteur de la HMG CoA réductase destiné à une utilisation selon l'une quelconque des revendications 1 à 11, dans lequel l'affection est une blépharite postérieure.

13. Composition formulée pour une administration ophtalmique, comprenant un inhibiteur de la HMG CoA réductase et comprenant en outre un composé anti-inflammatoire et/ou un immunosuppresseur, dans laquelle l'inhibiteur de la HMG CoA réductase est une statine, optionnellement dans laquelle l'inhibiteur de la HMG CoA réductase est l'atorvastatine.
